# EUROPEAN PATENT SPECIFICATION

(11) **EP 4 628 032 B1**
(45) Date of publication and mention of the grant of the patent: **21.10.2026**
(21) Application number: 23905930.6
(22) Date of filing: 19.12.2023
(51) Int. Cl.: A61B 17/072, A61B 17/00, A61B 17/29

(54) **END-EFFECTOR ASSEMBLY, INITIAL POSITION RETAINER, AND SURGICAL INSTRUMENT**
ENDEFFEKTORANORDNUNG, INITIALPOSITIONSHALTER UND CHIRURGISCHES INSTRUMENT
ENSEMBLE EFFECTEUR TERMINAL, DISPOSITIF DE RETENUE DE POSITION INITIALE ET INSTRUMENT CHIRURGICAL

(30) Priority: 19.12.2022 CN 202211632874
(43) Date of publication of application: 08.10.2025
(73) Proprietor: Reach Surgical, Inc., Tianjin 300462 (CN)
(72) Inventor: LI, Shuaishuai, Tianjin 300462 (CN)
(74) Representative: Sun, Yiming
(86) International application number: PCT/CN2023/139754
(87) International publication number: WO 2024/131767

(56) References cited:
- CN-A- 105 748 125
- CN-A- 110 477 984
- CN-A- 110 799 129
- CN-A- 115 153 702
- GB-A- 191 418 655
- US-A1- 2007 152 014
- US-A1- 2016 256 152
- US-B2- 10 729 509

## Description

### TECHNICAL FIELD

The present invention relates to the field of surgical instruments, and particularly relates to an end-effector assembly, an initial position retainer, and a surgical instrument for clamping, cutting, and stapling tissues.

### BACKGROUND

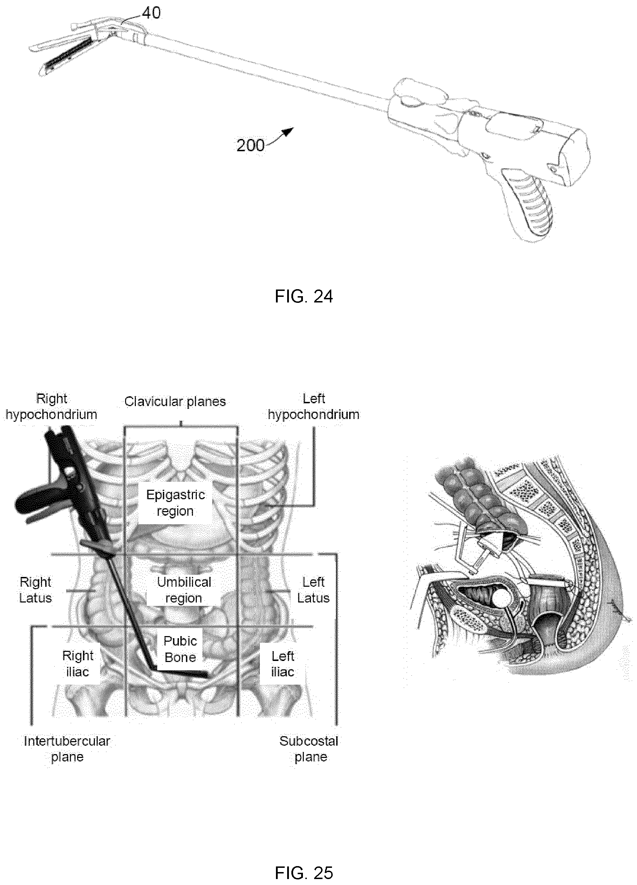

Laparoscopic surgical stapling instruments are commonly used surgical instruments in laparoscopic surgeries and are suitable for anastomosing and cutting tissues. Such instruments generally include a handle assembly, an elongated body assembly, and an end-effector assembly. During the surgery, a portion of the elongated body assembly and the end-effector assembly are advanced into the patient's body through a pathway created by a trocar device, and the doctor can operate the handle assembly to articulate the end-effector assembly to a certain angle relative to the elongated body assembly to adapt to different tissue cutting and anastomosis positions. In some specific laparoscopic surgical environments or procedures, it is often desirable that the end-effector assembly has a larger articulating angle. For example, surgeries remain the primary treatment for mid-to-low rectal cancer located 4-10 cm from the anal verge, with the main procedure being Low Anterior Resection (LAR). Due to human anatomical characteristics, the pelvic cavity is confined and rich in nerve plexuses and blood vessels. During LAR, the end effector assembly is required to provide a larger articulation angle and a smaller bending radius to facilitate a single-stroke transection of the rectum (refer to Fig. 25), thereby achieving a smaller incision and fewer stapling lines, reducing the probability of stapling leakage, and reducing the occurrence of postoperative complications.

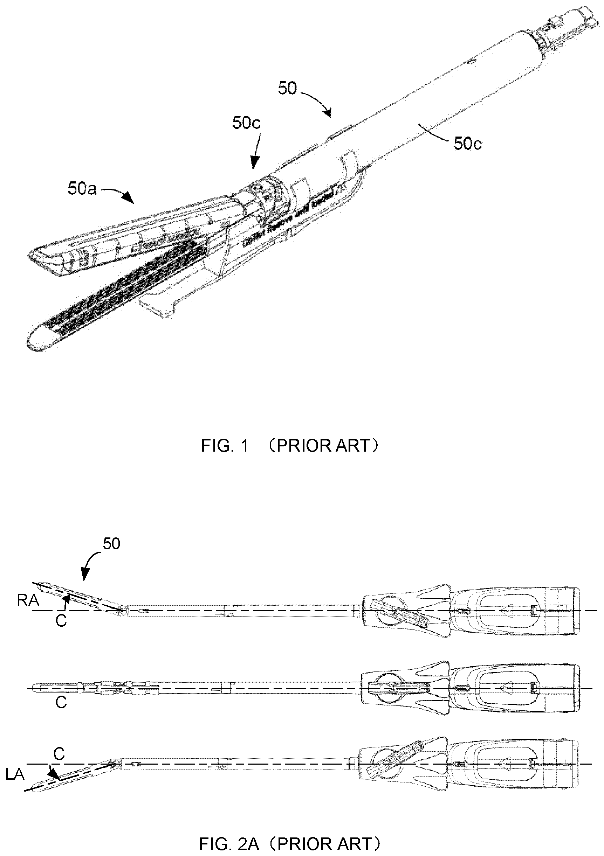

Referring to FIGS. 1-2, an end-effector assembly 50 in prior art includes a proximal body portion 50a and a distal execution portion 50b that are pivotally connected by means of an articulation joint 50c. The distal execution portion 50b can be articulated leftward (LA) or rightward (RA) to a certain angle relative to a longitudinal axis C defined by the proximal body portion 50a. Due to the structure of this symmetric articulation joint design, such as mechanical interference between components, the maximum articulation angle is typically limited to no more than 50 degrees articulation joint. Furthermore, end effectors in prior art achieving large articulation angles via dual or multi-joint mechanisms employ progressive bending around multiple pivot axes. In addition, although the end-effector assembly in prior art, which achieves large-angle articulating by means of dual or multi-joint mechanisms, has a larger articulating angle due to the use of multiple pivot pins for progressive articulating. While this provides a larger articulation angle, it simultaneously increases the length of the joint section and enlarges the bending radius of the end effector assembly. Consequently, it still cannot adapt to confined surgical environments such as the pelvic cavity.

CN110799129A discloses a surgical instrument comprising an end effector, a shaft, and an articulation joint. The shaft comprises a frame defining a longitudinal shaft axis and, in addition, a closure actuator translatable relative to the frame. The closure actuator comprises a proximal portion, a distal portion, and a link rotatably connected to the proximal and distal portions about proximal and distal link axes, wherein the proximal and distal link axes define a longitudinal link axis therebetween. The end effector is rotatably connected to the shaft about an articulation axis defined by the articulation joint, wherein the end effector is articulatable between an unarticulated position and an articulated position, wherein the articulation axis is offset from the longitudinal shaft axis, and the longitudinal link axis is not collinear with the longitudinal shaft axis when the end effector is in either the unarticulated position or the articulated position.

US10729509B2 discloses a surgical instrument assembly comprising an end effector, a shaft assembly, a closure drive system, and a firing drive system. The surgical instrument assembly further comprises a dual lock configured to engage a closure member and a firing member to prevent the firing member from being advanced distally from a proximal firing stroke position before the closure member is in a distal closure stroke position. The dual lock is further configured to prevent the closure member from being retracted from the distal closure stroke position before the firing member is returned to the proximal firing stroke position after the firing stroke.

US2016256152A1 discloses an end effector including first and second jaw members that are pivotable relative to one another between open and clamped configurations about a pivot axis. The first and second jaw members each define a pivot hole which defines the pivot axis. The first and second jaw members also each define an articulation axis that is orthogonal to the pivot axis and position distal of the pivot axis. The first and second jaw members are articulatable about the articulation axis between straight and fully articulated configurations.

### SUMMARY

The invention is defined in independent claim 1. Certain optional features of the invention are defined in the dependent claims.

To this end, the present invention provides an end-effector assembly of a surgical instrument and a surgical instrument adapted for achieving a large articulation angle.

In view of the above technical problem, the present invention is set out in appended claims.

The technical solution of the present invention has the following technical effects over the prior art:
in the surgical instrument and the end-effector assembly thereof provided in the present invention, the proximal body portion and the distal execution portion are pivotally connected through the articulation joint, and the articulation transmission member located on the proximal body portion acts on the articulation drive pin to provide a bending drive force to the articulation joint so that the distal execution portion bends relative to the longitudinal axis of the proximal body portion; and when the distal execution portion extends in the longitudinal axis direction, the articulation drive pin is located at the proximal end or the distal end of the pivot pin. This can increase the moving distance of the articulation transmission member, since the moving distance of the articulation transmission member is positively correlated with the articulation angle of the distal execution portion. The above-described structure of the present invention enables the distal execution portion to have an articulation angle ranging from 0° to 90°, and thus is particularly suitable for use in laparoscopic surgeries requiring an end-effector assembly to have a large articulation angle.

### BRIEF DESCRIPTION OF THE DRAWINGS

The preferred embodiments of the present invention will be described in detail below with reference to the drawings, which will help to understand the objects and advantages of the present invention, in which:
FIG. 1 is a schematic structural diagram of an end-effector assembly of a surgical instrument in the prior art;
FIG. 2A is a schematic structural diagram of three articulated states of the end-effector assembly of the surgical instrument in the prior art;
FIGS. 2B-C are diagrams showing a moving distance of an articulation transmission member in a longitudinal axis direction when the end-effector assembly of the surgical instrument in the prior art moves from an initial ready-to-be-loaded position to a maximum articulation angle;
FIG. 3 is a schematic structural diagram of a surgical instrument according to a specific embodiment of the present invention;
FIG. 4 is a schematic structural diagram of a main body portion of the surgical instrument according to a specific embodiment of the present invention;
FIG. 5 is a schematic diagram of a connection relationship between an end-effector assembly and the main body portion according to a specific embodiment of the present invention;
FIG. 6 is a schematic structural diagram of the end-effector assembly according to a specific embodiment of the present invention;
FIG. 7 is an exploded view showing some components of an elongated body assembly of the surgical instrument according to a specific embodiment of the present invention;
FIG. 8 is an exploded view showing some components of the elongated body assembly and a handle assembly of the surgical instrument according to a specific embodiment of the present invention;
FIG. 9 is an exploded view of the end-effector assembly according to a specific embodiment of the present invention;
FIG. 10 is an exploded view showing some structures of an articulation joint and a firing member in the end-effector assembly according to a specific embodiment of the present invention;
FIGS. 11A-B are schematic structural diagrams of a distal execution portion and a proximal body portion of the end-effector assembly that extend in a same direction according to a specific embodiment of the present invention;
FIGS. 12A-B are schematic structural diagrams of the end-effector assembly in an initial state (ready-to-be-loaded position) according to a specific embodiment of the present invention;
FIGS. 13A-B are schematic structural diagrams of the distal execution portion of the end-effector assembly at a maximum articulation angle according to a specific embodiment of the present invention;
FIG. 14A is another schematic structural diagram of the distal execution portion and the proximal body portion of the end-effector assembly that extend in a same direction according to a specific embodiment of the present invention;
FIG. 14B is another schematic structural diagram of the end-effector assembly in the initial state (ready-to-be-loaded position) according to a specific embodiment of the present invention;
FIG. 14C is another schematic structural diagram of the distal execution portion of the end-effector assembly at the maximum articulation angle according to a specific embodiment of the present invention;
FIG. 15A is a diagram showing a moving distance of an articulation transmission member in the longitudinal axis direction when the end-effector assembly changes from the initial state (ready-to-be-loaded position) to the maximum articulation angle according to a specific embodiment of the present invention;
FIG. 15B is a diagram showing a moving distance of the articulation transmission member in the longitudinal axis direction when the end-effector assembly changes from the initial state (ready-to-be-loaded position) to an unarticulated state according to a specific embodiment of the present invention;
FIG. 16A is a schematic structural diagram of the distal execution portion and the proximal body portion of the end-effector assembly that extend in a same direction according to another specific embodiment of the present invention;
FIG. 16B is a schematic structural diagram of the end-effector assembly in the initial state (ready-to-be-loaded position) according to another specific embodiment of the present invention;
FIG. 16C is a schematic structural diagram of the distal execution portion of the end-effector assembly at the maximum articulation angle according to another specific embodiment of the present invention;
FIG. 17A is a diagram showing a moving distance of the articulation transmission member in the longitudinal axis direction when the end-effector assembly changes from the initial state (ready-to-be-loaded position) to the maximum articulation angle according to another specific embodiment of the present invention;
FIG. 17B is a diagram showing a moving distance of the articulation transmission member in the longitudinal axis direction when the end-effector assembly changes from the initial state (ready-to-be-loaded position) to the unarticulated state according to another specific embodiment of the present invention;
FIG. 18 is a schematic structural diagram of an initial position retainer according to a specific embodiment of the present invention;
FIG. 19 is a top view of the initial position retainer according to a specific embodiment of the present invention;
FIG. 20 is a sectional view taken along line A-A in FIG. 19;
FIG. 21 is an assembly view of the end-effector assembly according to a specific embodiment of the present invention;
FIG. 22 is an enlarged view of portion A in FIG. 21;
FIG. 23 is a schematic structural diagram of a cartridge base and a staple anvil base in the end-effector assembly according to a specific embodiment of the present invention;
FIG. 24 is a schematic structural diagram of a surgical instrument according to another specific embodiment of the present invention; and
FIG. 25 is a schematic diagram of an LAR surgical procedure.

### DETAILED DESCRIPTION

The technical solutions of the present invention will be clearly and completely described below with reference to the drawings. Obviously, the described embodiments are part of rather than all of the embodiments of the present invention. All other embodiments obtained by those of ordinary skill in the art based on the embodiments in the present invention without creative efforts shall fall within the scope of protection of the present invention, which is determined by the appended claims.

In the description of the present invention, it should be noted that the orientation or positional relationships indicated by the terms "center", "upper", "lower", "left", "right", "vertical", "horizontal", "inner", "outer", and the like are based on the orientation or positional relationships shown in the drawings, and are only for the convenience of describing the present invention and simplifying the description, rather than indicating or implying that the device or element referred to must have a specific orientation, or be constructed and operated in a specific orientation, and therefore shall not be construed as a limitation to the present invention. In addition, the terms "first", "second", and "third" are used for descriptive purposes only, and shall not be construed as indicating or implying relative importance.

In the description of the present invention, it should be noted that, unless explicitly specified and defined otherwise, the terms "mount", "link", and "connect" should be interpreted in a broad sense. For example, they may refer to a fixed connection, a detachable connection, or an integral connection, or may refer to a direct connection, or an indirect connection via an intermediate medium, or may refer to an internal communication of two elements. The specific meanings of the above terms in the present invention may be understood by those of ordinary skill in the art in the specific context.

Furthermore, technical features referred to in different embodiments of the present invention described below may be combined with each other as long as they do not conflict with each other.

In various embodiments of the present invention, "distal end/side" refers to an end of a surgical instrument that is away from an operator when the surgical instrument is operated, and "proximal end/side" refers to an end/side of the surgical instrument that is close to the operator when the surgical instrument is operated.

The following is a specific embodiment of a surgical instrument. In general, the embodiment of the surgical instrument described herein relates to an endoscopic surgical cutting and anastomosis instrument. It should be noted, however, that the surgical instrument may also be a non-endoscopic surgical cutting and stapling instrument, such as an open surgical instrument for open surgeries.

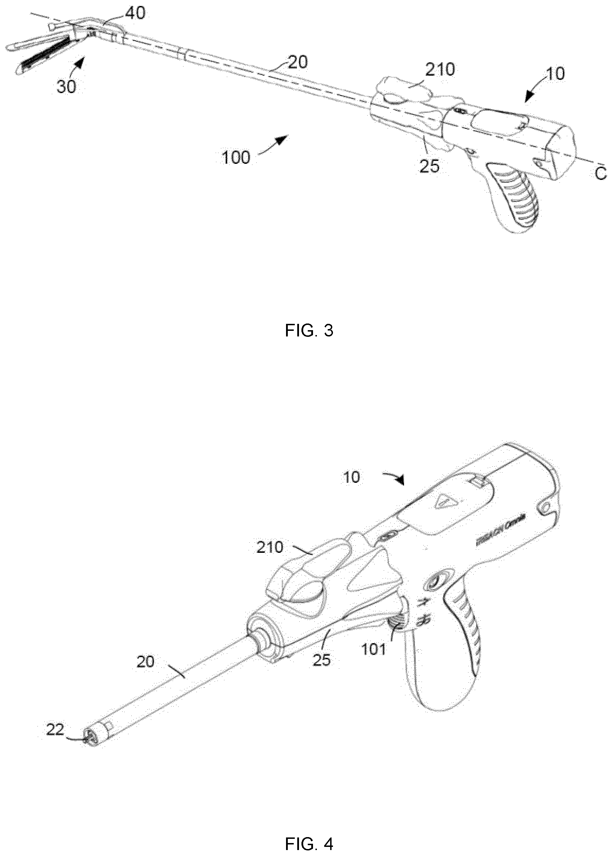

In particular, FIG. 3 illustrates an embodiment of a surgical instrument 100. The surgical instrument 100 includes a handle assembly 10, an elongated body assembly 20, and an end-effector assembly 30 arranged sequentially from proximal to distal, wherein the elongated body assembly 20 defines a longitudinal axis C and extends distally from the distal end of the handle assembly 10, and the end-effector assembly 30 is coupled with the distal end of the elongated body assembly 20 either detachably or non-detachably. The handle assembly 10 is adapted to allow an operator to manipulate the surgical instrument 100, and the handle assembly 10 may control the end-effector assembly 30 via the elongated body assembly 20 to perform surgical procedures, such as clamping/closing, suturing/stapling, cutting, etc. The handle assembly 10 includes a handle body that can be gripped by the operator in a conventional manner. In a specific embodiment, the surgical instrument 100 controls the closing and firing of the end-effector assembly by means of a trigger. Alternatively, the surgical instrument 100 can also control the closing and firing of the end-effector assembly by means of a push button, a button, or the like disposed on the handle body, so that the end-effector assembly 30 performs cutting and suturing operations. The elongate shaft assembly 20 is tubular and elongate, adapted to transmit driving force provided by the handle assembly to the distal end of the surgical instrument 100.

It should be noted that although the embodiments of the surgical instrument described herein are configured with an end effector assembly for cutting and stapling tissue, in alternative embodiments, other techniques for cutting and stapling tissue can be employed. For example, an end effector utilizing radiofrequency (RF) energy or adhesives to staple tissue could also be used.

The surgical instrument 100 described in the embodiment of the present invention further includes a rotation knob 25. The rotation knob 25 is mounted on the distal side of the handle assembly 10, and disposed at the proximal end of the elongated body assembly 20. The rotation knob 25, when being operated to rotate about the longitudinal axis C of the surgical instrument 100, can drive the elongated body assembly 20 and the end-effector assembly 30 to rotate together.

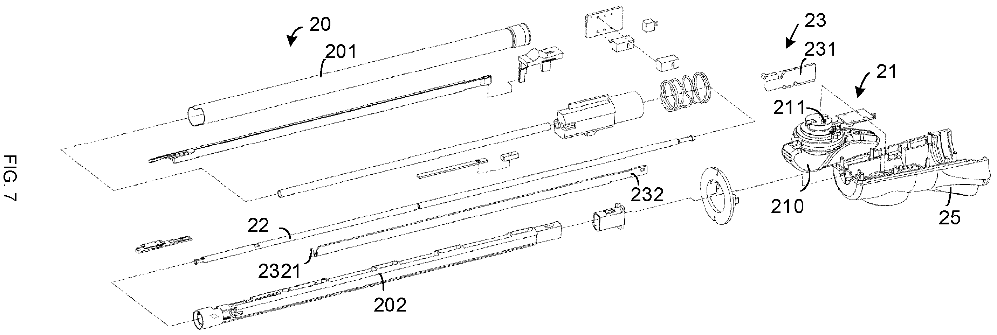

To achieve articulation of the end effector assembly 30 relative to the longitudinal axis C of the elongate shaft assembly 20 by a set angle, the end-effector assembly 30 includes a proximal body portion 30a and a distal execution portion 30b that are pivotally connected via an articulation joint 30c. Correspondingly, the surgical instrument 100 further includes an articulation drive assembly 21 and an articulation transmission assembly 23 for driving the articulation joint 30c to articulate. As shown in FIG. 7, the articulation transmission assembly 23 includes an articulation transmission frame 231 and an articulation link 232. The articulation transmission frame 231 is connected to the proximal end of the articulation link 232, and is operatively connected to the articulation drive assembly 21. The articulation drive assembly 21 includes an articulation knob 210 and an articulation drive member 211 which are mounted on the rotation knob 25. The articulation drive member 211 is connected to the articulation transmission frame 231. Rotation of the articulation knob 210 operated by the operator can drive the articulation drive member 211 to move. Specifically, when the articulation knob 210 is operated to rotate clockwise from an initial position, the articulation drive member 211 drives the articulation link 232 to move toward the distal end, and when the articulation knob 210 is operated to rotate counterclockwise from the initial position, the articulation drive member 211 drives the articulation link 232 to move toward the proximal end; and vice versa.

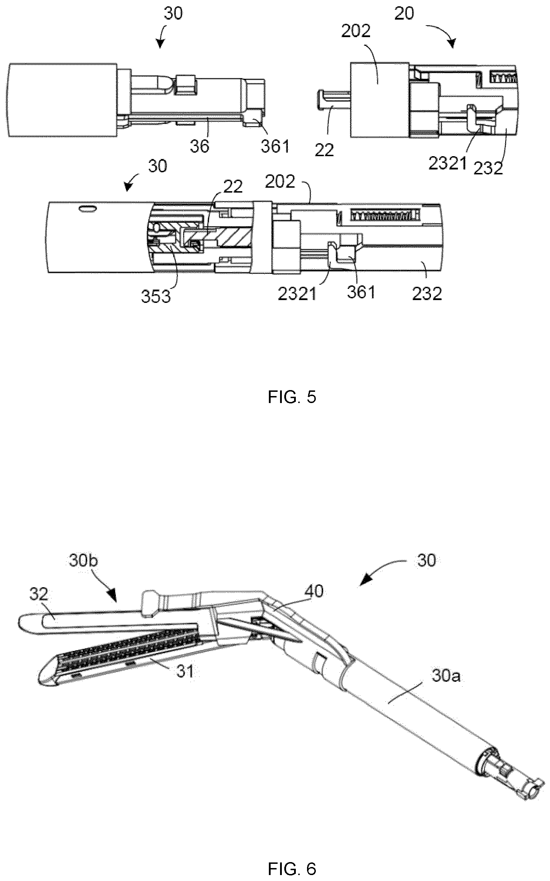

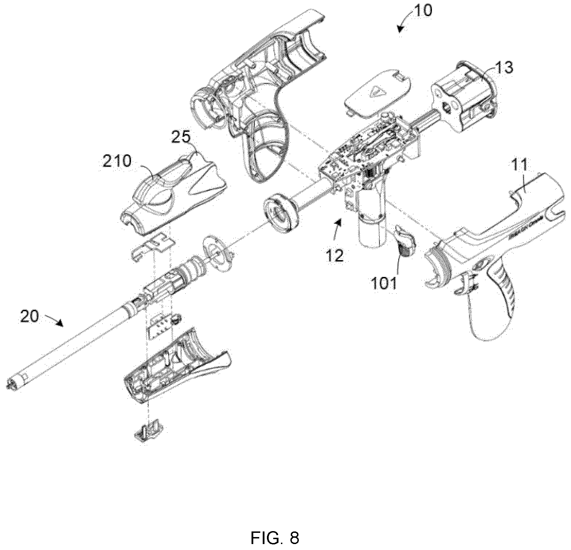

The surgical instrument 100 may also manipulate the closing and firing of the distal execution portion 30b via a button 101 disposed on the handle assembly 10, so that the distal execution portion 30b performs cutting and stapling operations on tissues. Specifically, as shown in FIGS. 7 and 8, the handle assembly 10 includes a drive mechanism 12 disposed in a handle housing 11 to provide output power for the surgical instrument 100. The elongated body assembly 20 includes an outer tube 201, a firing rod 22 is disposed in the outer tube 201, and the firing rod 22 is operably engaged with the drive mechanism 12 so as to be driven by the drive mechanism 12 to perform reciprocating movement along the longitudinal axis C, thereby achieving firing (advancing) and retracting functions of the surgical instrument 100. More specifically, the elongated body assembly 20 includes a support member 202 for slidably supporting the firing rod 22 and the articulation link 232, and the distal end of the support member 202 is provided with a tubular housing for engaging a proximal connecting end of the end-effector assembly 30. Referring to FIG. 5, a connecting piece 361 is disposed at the proximal end of an articulation transmission member 36 of the end-effector assembly 30 and fits with a connecting hook 2321 located at the distal end of the articulation link 232, and a connecting portion 353 is disposed at the proximal end of the firing member 35 in the end-effector assembly 30 and fits with the distal end of the firing rod 22, so as to realize rotational snap-fit engagement and locking between the end-effector assembly 30 and the elongated body assembly 20. In addition, the handle assembly 10 further includes a power supply 13 that provides electrical energy to the drive mechanism 12. The power supply 30 may be replaceable and/or rechargeable.

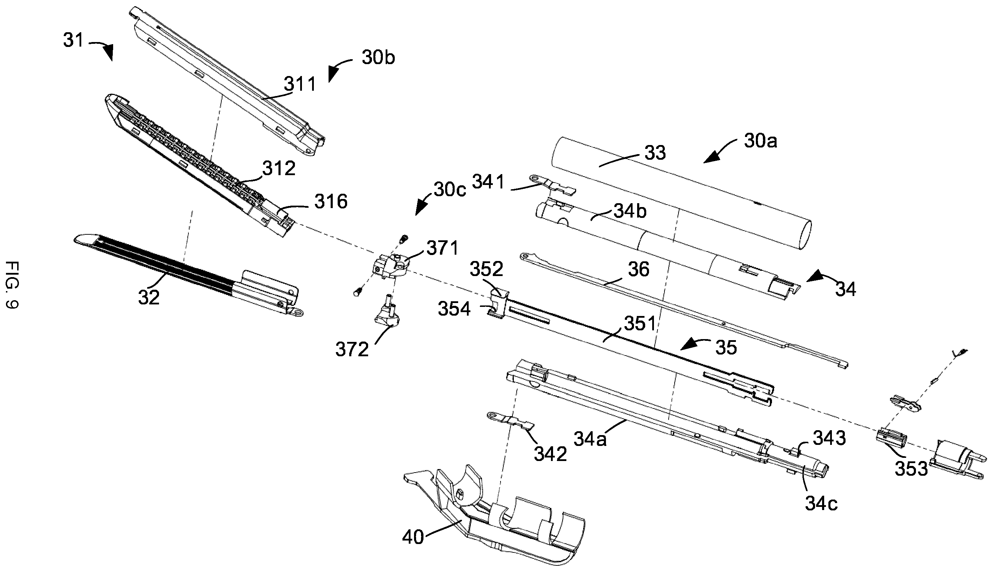

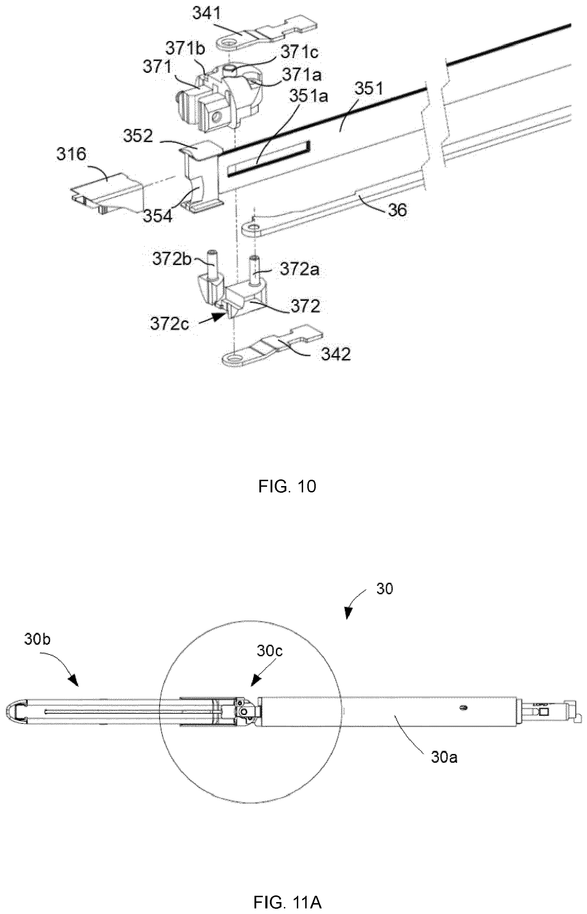

Further referring to FIGS. 9 and 10, a specific embodiment of the end-effector assembly 30 of the surgical instrument 100 of the present invention is described in detail. The end-effector assembly 30 is detachably mounted at the distal end of the elongated body assembly 20 of the surgical instrument 100. As previously described, the end-effector assembly 30 includes the proximal body portion 30a and the distal execution portion 30b that are pivotally connected via the articulation joint 30c. The proximal body portion 30a of the end-effector assembly 30 may be inserted into the elongated body assembly 20 by being plugged and rotated relative to a housing of the elongated body assembly 20 to lock the end-effector assembly 30 thereon. The staple cartridge assembly 31 and the anvil assembly 32 are movable relative to each other to close the jaws for grasping tissue. In one specific embodiment, the anvil assembly 32 can be operated to pivot toward the staple cartridge assembly 31 until the jaws of the end-effector assembly 30 are closed to clamp tissues; and the anvil assembly 32 pivots in a direction away from the staple cartridge assembly 31 until the jaws of the end-effector assembly 30 are opened to release tissues. In an alternative embodiment, the staple cartridge assembly 31 of the end-effector assembly 30 can be operated to pivot toward the anvil assembly 32 until the jaws of the end-effector assembly 30 are closed to clamp tissues; and the staple cartridge assembly 31 is operated to pivot in a direction away from the staple cartridge assembly 31 until the jaws of the end-effector assembly 30 are opened to release tissues.

The staple cartridge assembly 31 includes a cartridge base 311, a staple cartridge 312, and a drive sled 316 disposed in a cavity between the staple cartridge 312 and the cartridge base 311. Specifically, the cartridge base 311 is connected to the articulation joint 30c, and the staple cartridge 312, as a disposable component, is detachably connected within the cartridge base 311. In other alternative embodiments, the cartridge base 311 and the staple cartridge 312 in the staple cartridge assembly 31 may be fixedly connected or may be integrally formed as a component that is detachably connected to the elongated body assembly 20. The cartridge base 311 is formed as an open housing structure having a U-shaped or semi-circular cross-section, which is connected to the distal end of the elongated body assembly 20, and the staple cartridge 312 is inserted into the cartridge base 311 by means of engagement or the like. The staple cartridge 312 is shaped in the form of an elongated seat, one side surface thereof away from the cartridge base 311 is adapted for interacting with tissue, and a cutting blade groove extending from the proximal end to the distal end of the staple cartridge 312 is disposed on the side surface (also referred to as a staple forming surface) of the staple cartridge 312 that is used to interact with the tissue, and the cutting blade groove is suitable for a cutting blade 354 to pass therethrough to cut the tissue clamped between the contact surface and the anvil assembly 32 from the proximal end to the distal end of the staple cartridge 312. In an alternative embodiment, the cutting blade groove is located at an intermediate position on the upper surface of the staple cartridge 312, and the staple cartridge 312 is divided by the cutting blade groove into a first staple forming region and a second staple forming region. The first staple forming region and the second staple forming region of the staple cartridge 312 are respectively provided with at least two rows of staple openings, wherein the number of rows of the staple openings may be two, three, or more according to different surgical conditions. Each row of the staple openings is provided with several columns of openings, the openings are arranged in a longitudinal axis direction of the staple cartridge 312, the openings can be used to receive staples and staple drivers, and the staples are arranged on the respective sides of the staple drivers that face the staple forming surface of the staple cartridge 312. When the drive sled 316 is actuated to slide/move along the longitudinal axis, the staple drivers from the proximal side to the distal side sequentially emplace staples into tissues to achieve staple forming. The anvil assembly 32 includes an anvil cover 321 and an anvil plate disposed within the anvil cover 321. The surface of the anvil plate is provided with a plurality of staple forming pockets. The staple forming pockets correspond one-to-one with the positions of the staple openings on the staple cartridge 312. During tissue stapling, staples ejected from the staple openings contact the staple forming pockets.

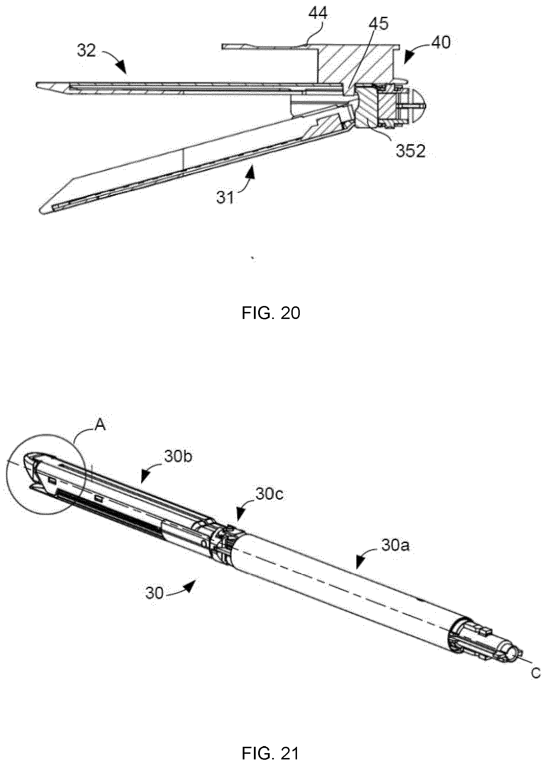

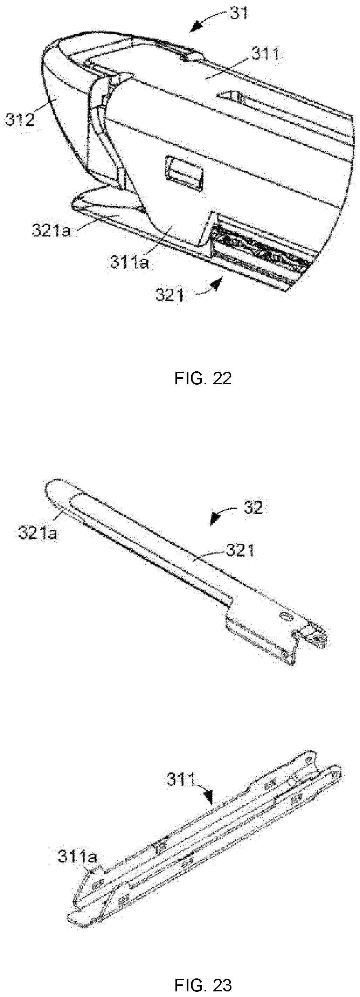

In order to prevent the staple cartridge assembly 31 and the anvil assembly 32 located at the distal end from slightly swinging in the width direction when the distal execution portion 30b is closed, resulting in a slight misalignment between the staple forming pockets in the anvil assembly and the staple openings in the staple cartridge 312, and ultimately resulting in a poor staple forming effect, the aforementioned end-effector assembly of the present invention, positioning structures fitting with each other are disposed at the distal ends of the staple cartridge assembly 31 and the anvil assembly 32, respectively, and the positioning structures are adapted to limit the lateral positions of the staple cartridge assembly 31 and the anvil assembly 32 in the width direction when the distal execution portion is in the closed position. Specifically, in an alternative embodiment, as shown in FIGS. 21-23, the positioning protrusions 311a are provided on two side walls of the cartridge base 311 of the staple cartridge assembly 31 in the thickness direction, positioning recesses 321a or notch structures are provided at corresponding positions of the anvil cover 321 of the anvil assembly, and the positioning protrusions 311a are inserted into the positioning recesses 321a to form a positioning structure to limit the position of the staple cartridge assembly 31 in the width direction, so that the two fit with each other at an accurate position and the suturing effect is good. In other alternative embodiments, the positioning protrusion is disposed on the anvil assembly 32 and the positioning recess is disposed on the staple cartridge assembly 31, and the fitting of the two also achieves the function of limiting the positions of the staple cartridge assembly 31 and the anvil assembly 32 in the width direction.

As further shown in FIG. 9, the proximal body portion 30a of the end-effector assembly 30 includes an elongated outer tube 33, an inner tube 34 disposed in the outer tube 33, a firing member 35 slidably connected to the inner tube 34, and the articulation transmission member 36. The inner tube 34 is formed by a first half-tube 34a and a second half-tube 34b, wherein the proximal end of the first half-tube 34a includes an engaging portion 34c for connecting to the elongated body assembly 20, and an engaging lug 343 is disposed on the engaging portion 34c for releasably engaging with the elongated body assembly 20 in a snap-fit connection manner. The first half-tube 34a and the second half-tube 34b define a sliding channel for slidably receiving the firing member 35. The firing member 35 includes an elongated firing beam 351, and the firing beam 351 may be formed of a single-sheet of deformable material, or preferably formed of a plurality of stacked sheets. A working portion 352 is provided at the distal end of the firing beam 351, and the cutting blade 354 is provided on the working portion 352 for forming an incision on a tissue to be cut during firing, wherein the working portion 352 of the firing member 35 is formed into an I-shaped beam structure, a partial region of which abuts against the slider 316 and the two can slide toward the distal end of the staple cartridge 312 as a whole to perform a corresponding surgical operation. For example, when the firing member 35 is driven to move from the proximal end to the distal end, a partial region of the working portion 352 of the firing member 35 pushes the slider 316 to move toward the distal end together, and the slider 316 acts on the staple drivers to push the staples out of the staple cartridge 312 to achieve the anastomosis operation on the tissue while the cutting blade 354 on the working portion 352 cuts the tissue. The connecting portion 353 is disposed at the proximal end of the firing beam 351. The connecting portion 353 is formed into a sleeve structure with an opening. A hole is provided at the proximal end of the connecting portion 353, and is configured to receive the distal end of the firing rod 22 when the proximal end of the end-effector assembly 30 is engaged with the elongated body assembly 20.

Referring to FIGS. 9 and 10, the articulation joint 30c includes a first link 371 and a second link 372, a pivot pin 371c is defined on the first link 371, the distal end of the inner tube 34 is fixedly connected to a pivotable support piece 341, and the distal end of the pivotable support piece 341 is pivotably connected to the pivot pin 371c, thereby pivotably connecting the articulation joint 30c to the proximal body portion 30a, and enabling the distal execution portion 30b to operably pivot relative to the proximal body portion 30a. In an alternative embodiment, in order to improve the stability of the pivotal movement of the distal execution portion 30b relative to the proximal body portion 30, corresponding coaxial pivot pins 371c, 372c are respectively disposed on the first link 371 and the second link 372, and pivotable support pieces 341, 342 are also disposed on the first half-tube 34a and the second half-tube 34b, respectively. The distal ends of the pivotable support pieces 341, 342 are pivotably connected to their corresponding pivot pins 371c, 372, respectively.

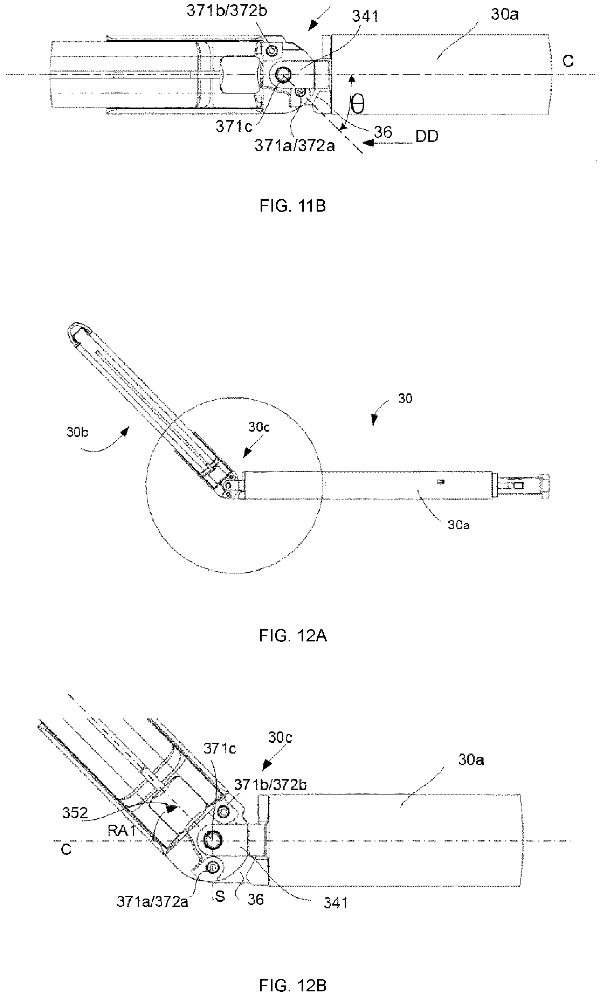

With continued reference to FIG. 10, the first link 371 and the second link 372 are connected by means of two connection pins 372a, 372b located on two opposite sides of the pivot pin 371c. Specifically, the connection pins 372a, 372b are fixedly provided on the second link 372, holes 371a, 371b corresponding thereto are disposed on the first link 371, and the connection pins 372a, 372b are fixedly connected to the holes 371a, 371b on the first link 372 by means of riveting, welding, or the like. Alternatively, in an alternative embodiment, each of the first link 371 and the second link 372 is provided with corresponding holes, and the first link 371 and the second link 372 are fixedly connected by riveting or welding two ends of each of the connection pins 372a, 372b, respectively. Further, the connecting shaft 372a of the articulation joint 30c is also adapted for fitting with a hole at the distal end of the articulation transmission member 36, so as to convert movement along the longitudinal axis C provided by the articulation transmission member 36 into a pivotal movement of the articulation joint 30c about the pivot pin 371c and/or the pivot pin 372c. In order to describe the specific technical solution of the present invention more clearly, the connecting shaft 372a engaged with the articulation transmission member 36 is hereinafter referred to as an articulation drive pin 372a. The distal end of the articulation transmission member 36 in the proximal body portion 30a is pivotably connected to the articulation drive pin 372a. Next, an articulation process of the end-effector assembly 30 provided in the embodiments of the present invention will be described in detail with reference to FIGS. 11-15.

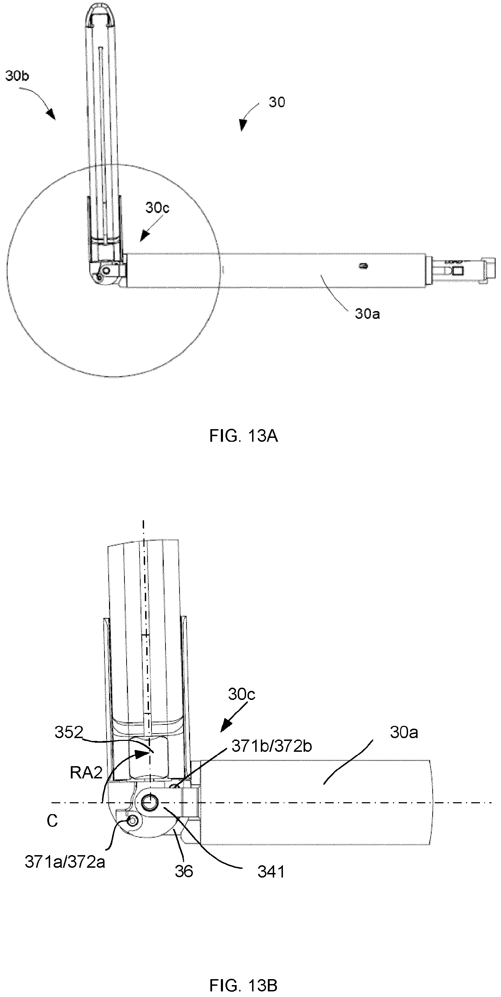

As shown in FIGS. 11A and 11B, when the distal execution portion 30b extends along the longitudinal axis C, that is, when the distal execution portion 30b extends in the same direction as the proximal body portion 30a, an included angle formed by the distal execution portion 30b and the longitudinal axis C is 0° or approximately 0°, and the articulation drive pin 372a is located at the proximal end of the pivot pin 371c. The articulation knob 210 of the surgical instrument 100 is operated so that the articulation transmission member 36 is actuated to move distally (in the direction of an arrow DD), driving the articulation drive pin 372a to pivot about the pivot pin 371c, and in such a way, the distal execution portion 30b is articulated in a direction gradually away from the longitudinal axis C, and finally reaches a position/state of a maximum articulating angle at one side, as shown in FIGS. 13A and 13B. During the process of the distal execution portion 30b articulating in the direction away from the longitudinal axis C, when a certain included angle is reached between the distal execution portion 30b and the longitudinal axis C, as shown in FIGS. 12A and 12B, the end-effector assembly 30 according to the embodiments of the present invention is in an unloaded or unused initial state/position (hereinafter referred to as a ready-to-be-loaded position). The end-effector assembly 30, when in the ready-to-be-loaded position, may be coupled and plugged into the distal end of the elongated body assembly 20 of the surgical instrument 100.

It can be understood that the maximum articulation angle that can be provided by the end-effector assembly, in addition to being limited by the mechanical interference of related parts in the end-effector assembly, is also limited by a maximum stroke that can be provided by the articulation link 232 in the handle assembly and the elongated body assembly of the surgical instrument.

To enable the end effector assembly 30 of the embodiments of the present invention to be compatible with the main body portion (including the handle assembly and the elongated body assembly) of the surgical instrument in the prior art, i.e., to allow the end effector assembly 30 of the present invention to be compatible with the same main body portion (handle assembly and elongate shaft assembly) as a prior art end effector assembly 50 (refer to FIG. 1) of the same specification and size, in one specific embodiment, with reference to FIGS. 12A and 12B, when the end-effector assembly 30 is in the ready-to-be-loaded position, and a line S connecting the axis center of the articulation drive pin 372a and the axis center of the pivot pin 371c of the articulation joint 30c is perpendicular to the longitudinal axis C, a predetermined first included angle RA1 is formed between the distal execution portion 30b and the longitudinal axis C.

Specifically, after the end-effector assembly 30 in the ready-to-be-loaded position is arranged on the elongated body assembly 20 of the surgical instrument 100, the articulation link 232 is caused to move distally by a first distance by operating the articulation knob 210 of the handle assembly 10, and correspondingly, the articulation transmission member 36 is also driven to move distally by L1, so that the distal execution portion 30b of the end-effector assembly 30, which has been articulated through the first angle RA1, is further articulated away from the longitudinal axis C to form a second included angle RA2. Similarly, after the end-effector assembly 30 in the ready-to-be-loaded position is arranged on the elongated body assembly 20 of the surgical instrument 100, the articulation link 232 is caused to move proximally by a second distance by operating the articulation knob 210 of the handle assembly 10, and correspondingly, the articulation transmission member 36 is also driven to move proximally by L2, so that the distal execution portion 30b of the end-effector assembly 30, which has been articulated through the first included angle RA1, further pivots in a direction close to the longitudinal axis C and ultimately forms an angle of 0° or substantially 0° relative to the longitudinal axis C for better passage of the end-effector assembly 30 through a trocar device, wherein the first distance and the second distance may be the same or different.

When the end-effector assembly 30 according to the embodiments of the present invention is adapted to the prior art surgical instrument, the first distance may correspond to a distance moved by the articulation link when the end-effector assembly 50 is articulated rightward to the maximum angle RA, and the second distance may correspond to a distance moved by the articulation link when the end-effector assembly 50 is articulated leftward to the maximum angle LA. The arrangement of the end-effector assembly 30 further broadens the applicability and adaptability of the end-effector assembly according to the embodiments of the present invention.

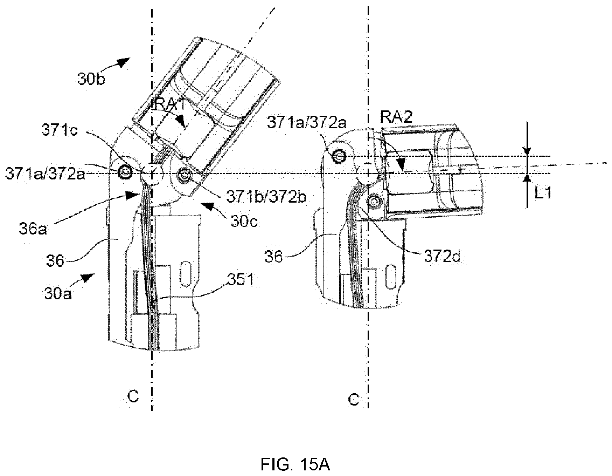

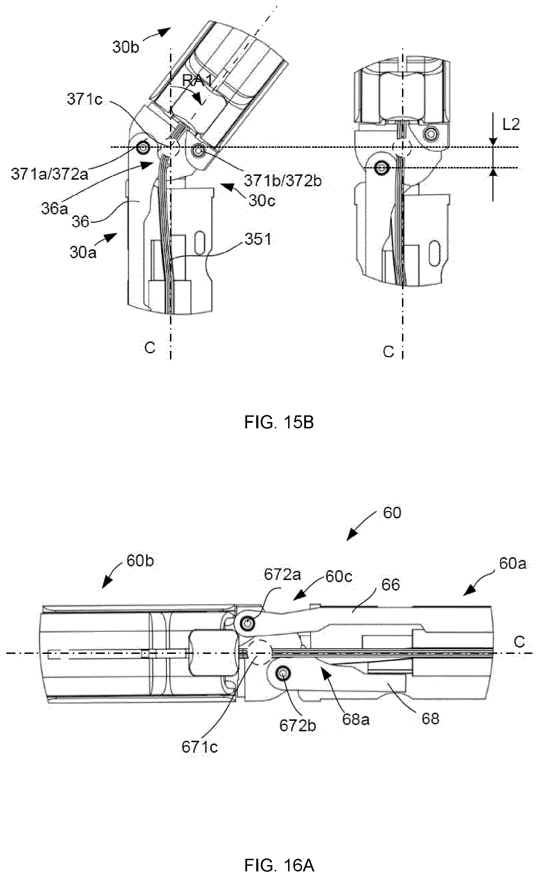

In one specific embodiment, referring to FIG. 15A, when the articulation knob 210 is operated to rotate clockwise from an initial position to a maximum rotatable position, the articulation link 232 is driven to move distally by the first distance, then the articulation drive pin 372a is driven to move distally by L1 along the longitudinal axis C, and the distal execution portion 30b that has been articulated through the first included angle RA1 is further articulated to the maximum articulation angle RA2. Referring to FIG. 15B, when the articulation knob 210 is operated to rotate counterclockwise from the initial position to the maximum rotatable position, the articulation drive member 211 drives the articulation link 232 to move proximally by the second distance, then the articulation drive pin 372a is driven to move proximally by L2 along the longitudinal axis C, and the distal execution portion 30b that has been articulated through the initial first included angle RA1 is articulated to extend in the same direction as the longitudinal axis C, that is, the articulation angle is 0° or approximately 0°. It can be seen that when the articulation transmission member 36 moves distally by L1+L2 in the longitudinal axis C direction, or in other words, the articulation transmission member 36 is operated to move distally along the longitudinal axis C from a proximal start position to a distal end position, the included angle formed by the distal execution portion 30b and the longitudinal axis C ranges from 0° or approximately 0° to the maximum articulation angle RA2.

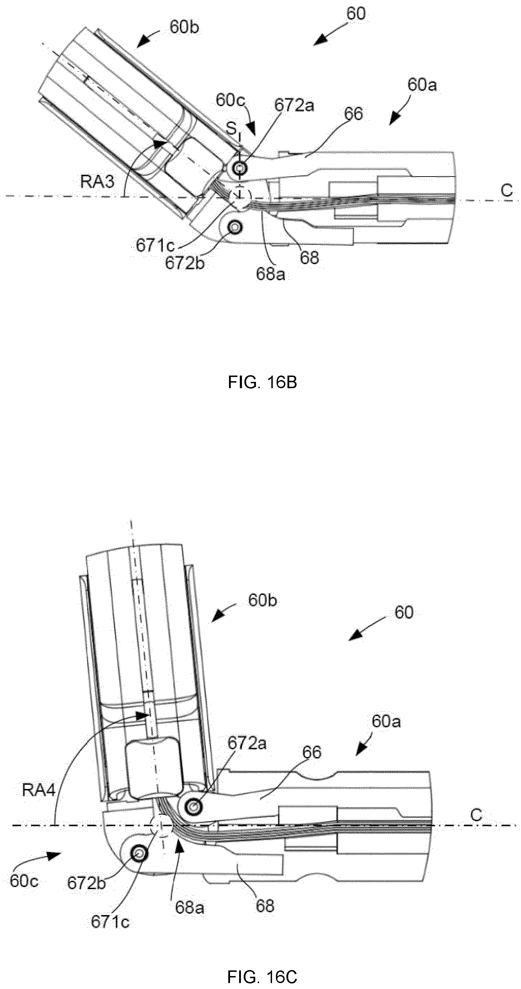

As shown in FIGS. 16-17, in an alternative embodiment, the articulation drive pin 672a of the end-effector assembly 60 is pivotably connected to the distal end of the articulation transmission member 66. Articulation of the distal effector portion 60b and the articulation joint 60c relative to the proximal body portion 60a is achieved by the articulation transmission member 66 being operably pulled proximally. Specifically, referring to FIG. 16A, when the angle between the distal effector portion 60b and the longitudinal axis C defined by the proximal body portion 60a is 0° or approximately 0°, the articulation drive pin 672a is located distal to the pivot pin 671c. The articulation knob 210 is operated to cause the articulation transmission member 66 to move proximally, thereby pulling the articulation drive pin 672a to pivot about the pivot pin 671c and driving the distal execution portion 60b to gradually move away from the longitudinal axis C until it is articulated to a maximum angle, referring to FIG. 16C. Similarly, in order to enable the end-effector assembly 60 to be adapted to the main body portion (including the handle assembly and the elongated body assembly) of the prior art surgical instrument and to fully utilize a maximum stroke that the articulation link 232 can provide, the state/position of the end-effector assembly 60 shown in FIG. 16B is defined as an unloaded or unused initial state/position (hereinafter referred to as a ready-to-be-loaded position). At this point, a line S connecting the axis centers of the articulation drive pin 672a and the pivot pin 671c of the end-effector assembly 60 is perpendicular to the longitudinal axis C.

After the end-effector assembly 60 in the ready-to-be-loaded position is arranged on the elongated body assembly 20 of the surgical instrument 100, the articulation link 232 is caused to move proximally by a third distance by operating the articulation knob 210 of the handle assembly 10, and correspondingly, the articulation transmission member 66 is also driven to move proximally by L3 so that the distal execution portion 60b of the end-effector assembly 60 which has been articulated through a third included angle RA3 is further articulated away from the longitudinal axis C until a fourth included angle RA4 is formed. Similarly, after the end-effector assembly 60 in the ready-to-be-loaded position is mounted on the elongated body assembly 20 of the surgical instrument 100, the articulation link 232 is caused to move distally by a fourth distance by operating the articulation knob 210 of the handle assembly 10, and correspondingly, the articulation transmission member 66 is also driven to move distally by L4 so that the distal execution portion 60b of the end-effector assembly 60 which has been articulated through the third included angle RA3 further pivots in a direction close to the longitudinal axis C, and finally forms 0° or substantially 0° with the longitudinal axis C for better passage of the end-effector assembly 60 through the trocar device, wherein the third distance and the fourth distance may be the same or different.

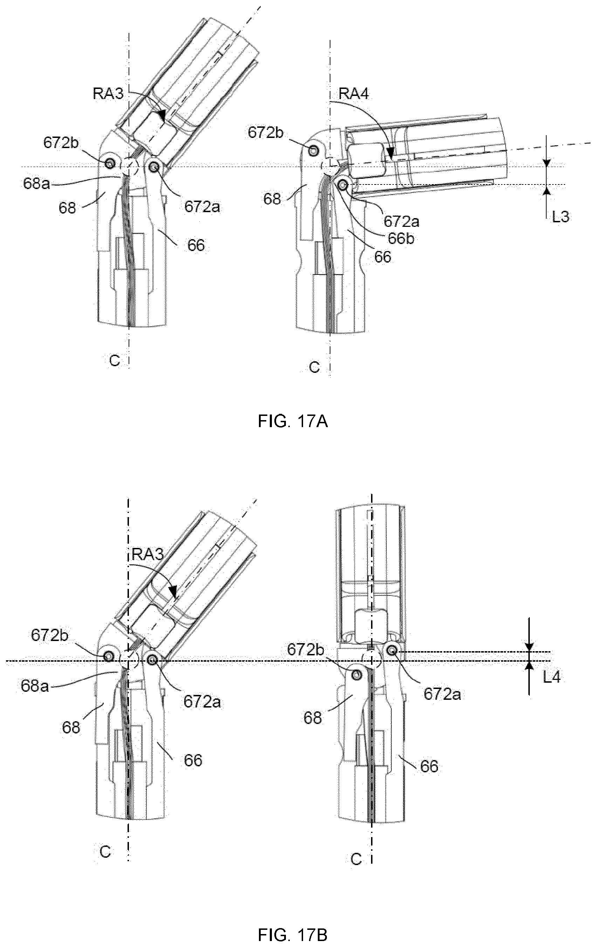

In a specific embodiment, referring to FIG. 17A, when the articulation knob 210 is operated to rotate clockwise from the initial position to the maximum rotatable position, the articulation link 232 is driven to move proximally by the third distance, then the articulation drive pin 372a is driven to move proximally by L3 along the direction of the longitudinal axis C, and the distal execution portion 30b is articulated to the maximum articulation angle. When the articulation knob 210 is operated to rotate counterclockwise from the initial position to the maximum rotatable position, the articulation drive member 211 drives the articulation link 232 to move distally by the fourth distance, then the articulation drive pin 372a is driven to move distally by L4 along the direction of the longitudinal axis, and the distal execution portion 30b is operated to extend in the same direction as the longitudinal axis C, that is, the articulation angle is 0° or approximately 0°. It can be seen that when the articulation transmission member 36 is actuated to move proximally by L3 + L4 along the direction of the longitudinal axis C, or in other words, the articulation transmission member 36 is operated to move proximally along the direction of the longitudinal axis C from the distal start position to the proximal end position, and the included angle formed by the distal execution portion 30b and the longitudinal axis C ranges from 0° or approximately 0° to the maximum articulation angle RA4.

Of course, it can be understood that, on the basis of the concept of the present invention, the end position of the articulation transmission member 36, 66 is not limited to the position corresponding to the position where the distal execution portion 30b extends along the longitudinal axis C, that is, the position where the included angle formed by the distal execution portion 30b and the longitudinal axis C is 0° or approximately 0°, but may also form a small included angle with the longitudinal axis C according to actual scenario requirements, whether it is articulated left or right.

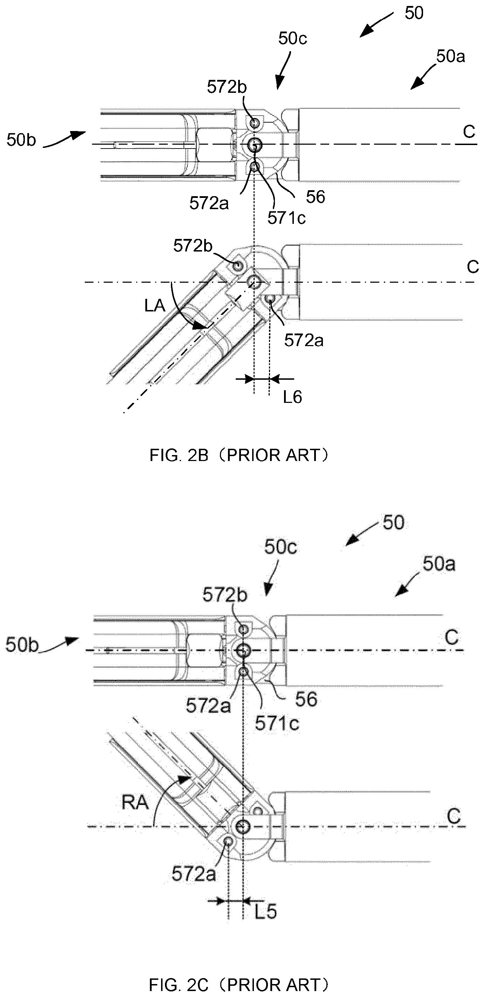

Referring to FIGS. 2B-C, in the end-effector assembly 50 of the prior art, when the articulation knob 210 is operated to rotate clockwise from the initial position to the maximum rotatable position, the articulation link 232 is driven to move distally by a fifth distance, then the articulation transmission member 56 and the articulation drive pin 572a are driven to move distally by L5 along the direction of the longitudinal axis C, and the distal execution portion 50b is articulated clockwise to the maximum articulation angle RA. Referring to FIG. 2C, when the articulation knob 210 is operated to rotate counterclockwise from the initial position to the maximum rotatable position, the articulation link 232 is driven to move proximally by a sixth distance, then the articulation transmission member 56 and the articulation drive pin 572a are driven to move proximally by L6 in the longitudinal axis C direction, and the distal execution portion 50b is articulated counterclockwise to the maximum articulation angle LA. It can be seen that when the articulation transmission member 56 is actuated to move along the direction of the longitudinal axis C by L5 or L6, the included angle formed by the distal execution portion 50b and the longitudinal axis C ranges from 0° to the maximum articulation angle RA, LA. As can be seen, when the articulation transmission member 56 is actuated to move proximally by L5 + L6 along the direction of the longitudinal axis C from the distal-most position, the included angle formed by the distal execution portion 50b and the longitudinal axis C ranges from a maximum articulation angle RA on one side to a maximum articulation angle LA on the other side.

It can be seen that when the maximum movable distances of the articulation transmission members are the same, the structure of the end-effector assembly 30, 60 according to the present invention can provide a larger maximum articulation angle than the structure of the prior art end-effector assembly 50.

In any embodiment of the end effector assembly 30, 60 of the present invention, when the included angle formed by the distal execution portion 30b, 60b and the longitudinal axis C is 0°, the line connecting the axis center of the articulation drive pin 372a, 672a and the axis center of the pivot pin 371c, 671c forms an angle θ with the longitudinal axis C, and the magnitude of θ is one of the factors that determine the maximum articulation angle provided by the end-effector assembly 30, 60. Accordingly, when the end-effector assembly 30, 60 is in the ready-to-be-loaded position, the articulation angle of the distal execution portion 30b, 60b is (90-θ)°. It can be understood that the smaller the value of θ, the greater the maximum articulation angle that can be provided by the end-effector assembly 30, 60, without considering the mechanical interference of parts and the maximum strokes that can be provided by the articulation drive assembly/articulation transmission assembly. For example, in a specific embodiment, when the included angle formed by the distal execution portion 30b, 60b and the longitudinal axis C is 0°, the included angle θ formed by a line connecting the shaft centers of the articulation drive pin 372a, 672a and the pivot pin 371c, 671c and the longitudinal axis C is 45°, which allows the maximum articulation angle of the distal execution portion 30b, 60b to be about 90°.

When the distal execution portion 30b, 60b is articulated to a large articulation angle relative to the proximal body portion 30a, 60a, in order to enable the firing member 35 to be accordingly bent and deformed and smoothly perform a firing operation, the end-effector assembly 30, 60 further includes a constraint channel for limiting movement of the firing beam during firing of the firing member 35 when the distal execution portion 30b, 60b is in an articulated state, the constraint channel is arranged in a fitting region between the proximal body portion 30a, 60a and the articulation joint 30c, 60c, and the firing beam 351 of the firing member 35 is received in the constraint channel and moves in a direction in which the constraint channel extends. The constraint channel is formed by a first constraint portion and a second constraint portion located on opposite sides of the firing beam 351, the first constraint portion is adapted to be engaged with the outer convex arc surface of the firing beam 351 when being most articulated, and the second constraint portion is adapted to be engaged with the inner arc surface of the firing beam 351 when being most articulated.

In any embodiment of the end-effector assembly 30 described in the present invention, as shown in FIG. 15A, the first constraint portion is designed as a first constraint protrusion 36a disposed on the articulation transmission member 36, and the first constraint protrusion 36a has an concave arc surface that matches the outer arc surface of the firing beam 351 when being most articulated(i.e., an outer arc surface of a bending region of the firing beam 351 when the firing beam 351 is articulated to its maximum articulation angle in FIG. 15A). Specifically, the articulation transmission member 36 is formed into an elongated sheet, the first constraint protrusion 36a is formed in a partial region of the distal end of the articulation transmission member 36 and protrudes toward the firing member 35, a side surface of the first constraint protrusion 36a facing the firing beam 351 forms an concave arc surface, and an arc length of the concave arc surface covers a maximum deformation region of the firing beam 351. The second constraint portion is a second constraint protrusion 372d disposed on the articulation joint 30c, and the second constraint protrusion 372d has an convex arc surface that matches the inner arc surface of the firing beam 351 when being most articulated (i.e., an inner arc surface of a bending region of the firing beam 351 when the firing beam 351 is articulated to its maximum articulation angle). Specifically, the second link 372 of the articulation joint 30c is provided with a second constraint protrusion 372d near an inner curved region of the firing beam 351, the second constraint protrusion 372d has an convex arc surface region, the constraint channel is formed between the articulation transmission member 36 and the second link 372 to limit the articulation position of the firing beam 351, and the firing beam 351 is bent along the constraint channel, preventing it from dislocating sideways during movement of being articulated to high articulation angles. In this embodiment, the articulation transmission member 36 receives articulation driving force directed distally. When driven by the articulation drive mechanism to slide from proximal to distal, the first constraint protrusion 36a of the articulation transmission member 36 moves distally into the region of the articulation joint 30c, cooperating with the second constraint protrusion 372d on the articulation joint 30c to form a constraint channel with an approximately 90-degree bend angle.

In any embodiment of the end-effector assembly 60 described in the present invention, as shown in FIGS. 16A-C, the first constraint portion includes a constraint member 68 disposed on the proximal body portion 60a, and the constraint member 68 has a concave arc surface 68a that matches the outer arc surface of the firing beam when being most articulated. Specifically, the proximal end of the constraint member 68 is fixedly connected to an inner tube of the proximal body portion 60a and the distal end is sleeved on the connecting shaft 672b, and a side thereof close to the firing beam is formed as the concave arc surface 68a, wherein an arc length of the concave arc surface 68a covers a maximum deformation region of the firing beam. The second constraint portion is a convex arc surface 66b disposed at the distal end of the articulation transmission member 66, and the convex arc surface 66b matches the inner arc surface of the firing beam when being most articulated.

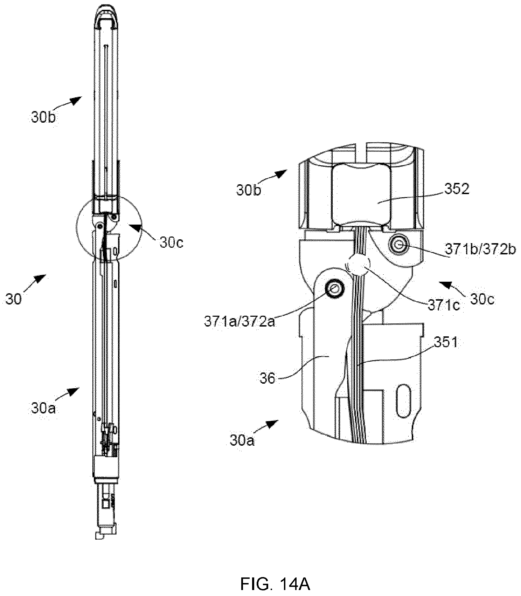

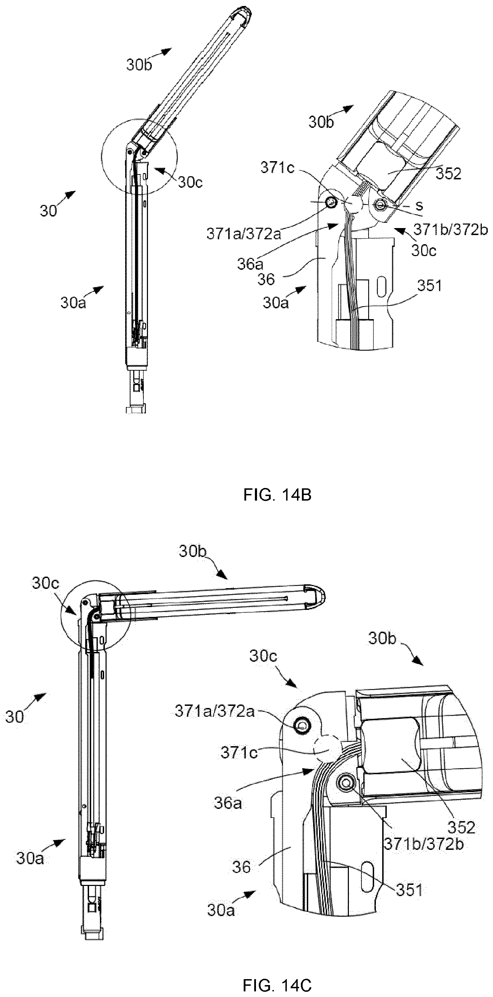

As shown in FIG. 10, a receiving slot 351a is provided in the distal portion of the firing beam 351 of the firing member 35, and when the articulation angle of the distal execution portion 30b is less than a set value, as shown in FIGS. 14A and 16A, a partial region of the first constraint portion is received in the receiving slot 351a.

In any of the above-described embodiments of the present invention, when the distal execution portion 30 is operated to extend along the direction of the longitudinal axis C, the articulation drive pin 371a, 372a is located proximal or distal to the pivot pin 371c, 372c. The distal execution portion 30b is operated to articulate unidirectionally away from the longitudinal axis C, articulatable between a unarticulated (or straight) position (i.e., a position where the included angle formed by the distal execution portion 30b, 60b and the longitudinal axis C is 0° or approximately 0°), an intermediate articulated position, and a most articulated position, enabling large-angle unidirectional articulation of the end effector assembly of the surgical stapling instrument. Furthermore, the end effector assembly 30, 60 provided by any embodiment of the present invention still employs a single pivot pin articulation method, ensuring the smallest possible bend radius, or in other words, increasing the maximum articulation angle achievable by the end effector assembly without increasing its bend radius.

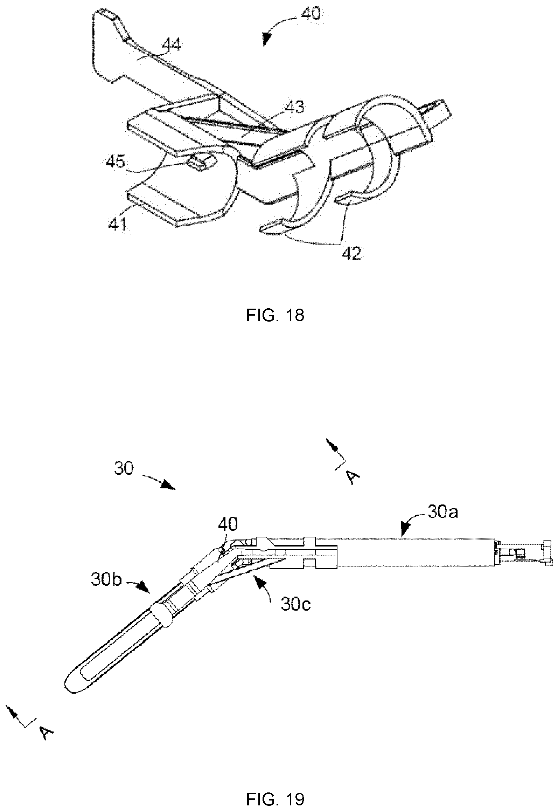

Since the ready-to-be-loaded position of the end-effector assembly 30, 60 provided in any embodiment of the present invention is a position where the distal execution portion 30b, 60b forms a certain included angle θ with the longitudinal axis C, the present invention further provides an initial retainer so that the end-effector assembly 30, 60 is more reliably retained in the ready-to-be-loaded position. As shown in FIGS. 18 to 20, this is a specific embodiment of the initial position retainer 40 according to the present invention. The initial position retainer 40 has a support body having a set angle so that the distal execution portion 30b, 60b of the end-effector assembly 30, 60 is retained in an initial articulated state for loading. Specifically, the initial position retainer 40 is used to support the distal execution portion 30b and the proximal body portion 30a of the end-effector assembly 30 so that the distal execution portion 30b is retained in an initial articulated state at a set angle relative to the proximal body portion 30a. Specifically, the support body includes a first holding arm 41 being engaged with the proximal body portion 30a and a second holding arm 42 being engaged with the distal execution portion 30b, and further includes a supporting beam 493 connected between the first holding arm 41 and the second holding arm 42, wherein the first holding arm 41 and the second holding arm 42 are formed in a half-ring shape that is semi-open and matches the shapes of the proximal body portion 30a and the distal execution portion 30b, which are held on the two to prevent separation.

More specifically, in order to facilitate gripping by a user, the initial position retainer 40 further includes a grip frame 44 suitable for the operator to grip, the grip frame 44 is connected to the outer side wall of the support body, and the structure of the grip frame 44 is not unique. In an alternative manner, the grip frame is formed into a hand-grip member suitable for the hand to pass through, and the hand-grip member runs through the entire longitudinal side wall of the support body and extends to an outer side region of the support body to form a cantilever structure. More specifically, the inner wall of the second holding arm 42 of the initial position retainer 40 is provided with a position-limiting structure for fitting with the working portion 352 of the firing member 35. As shown in FIGS. 18-20, the position-limiting structure is a protrusion structure 45 formed on the inner wall of the second holding arm 42, and the protrusion structure 45 extends into the staple anvil base along a through-hole of the staple anvil base 321 and abuts against the working portion 352 of the firing member 35 to prevent the firing member 35 from moving toward the distal end.

It can be understood that the end-effector assembly and the initial position retainer provided according to any embodiment of the present invention, in addition to being applicable to universal surgical stapling instruments, that is, the entire end-effector assembly is detachably mounted to an elongated body assembly of a surgical instrument, for example, the surgical instrument 100 provided according to any embodiment of the present invention, may be applicable to fixed-fit surgical stapling instruments, that is, a proximal body portion of the end-effector assembly is non-detachably fixed to an elongated body assembly of a surgical instrument, while portions of a staple cartridge assembly are detachably replaceable, for example, the surgical instrument 200 shown in FIG. 24.

Obviously, the above-described embodiments are merely examples for clarity of description and are not intended to limit the embodiments. For those of ordinary skill in the art, other different forms of variations and modifications can be made based on the above description. It is not necessary and impossible to list all embodiments here. Obvious variations and modifications derived therefrom are within the scope of protection of the present invention, which is determined by the appended claims.

## Claims

1. An end-effector assembly (30, 60), comprising:
a proximal body portion (30a, 60a) that defines a longitudinal axis (C); and
a distal execution portion (30b, 60b) adapted to manipulate tissue, wherein the distal execution portion being pivotally connected to the proximal body portion through an articulation joint (30c, 60c) provided with a pivot pin (371c) thereon;
the proximal body portion comprises an articulation transmission member (36, 66), and an articulation drive pin (372a, 672a) is arranged on the articulation joint, wherein the articulation transmission member is pivotably connected to the articulation drive pin, and the articulation transmission member is operable to perform reciprocating movement along the direction of the longitudinal axis to actuate the articulation drive pin to pivot about the pivot pin and to actuate the distal execution portion to pivot about the pivot pin; and
when the end-effector assembly is in a ready-to-be-loaded position, the distal execution portion forms an included angle not equal to 0° relative to the longitudinal axis;
**characterized in that**
the end-effector assembly further comprises a constraint channel adapted to limit an articulation position of a firing member (35) during a firing motion when the distal execution portion is in an articulated state, the constraint channel being arranged in a fitting region between the proximal body portion and the articulation joint, and the firing member being arranged in the constraint channel and movable in a direction in which the constraint channel extends.

2. The end-effector assembly according to claim 1, wherein when the distal execution portion is operated to extend along the direction of the longitudinal axis, a line connecting an axis center of the articulation drive pin and an axis center of the pivot pin forms an angle not equal to 0° with respect to the longitudinal axis.

3. The end-effector assembly according to claim 1, wherein when the distal execution portion is operated to extend along the direction of the longitudinal axis, the articulation drive pin is located proximal or distal to the pivot pin.

4. The end-effector assembly according to claim 1, wherein when the end-effector assembly is in the ready-to-be-loaded position, the articulation joint is pivoted so that a line connecting an axis center of the articulation drive pin and an axis center of the pivot pin is perpendicular to the longitudinal axis.

5. The end-effector assembly according to claim 1, wherein when the articulation transmission member is operated to move from a start position to an end position, the distal execution portion is actuated to pivot about the pivot pin and to be gradually articulated away from the longitudinal axis.

6. The end-effector assembly according to claim 5, wherein when the articulation transmission member is in the start position, the distal execution portion is operated to extend along the direction of the longitudinal axis.

7. The end-effector assembly according to claim 5, wherein when the end-effector assembly is in the ready-to-be-loaded position, the articulation transmission member is located in an intermediate position that is between the start position and the end position.

8. The end-effector assembly according to claim 1, wherein the constraint channel is formed by a first constraint portion and a second constraint portion arranged on opposite sides of the firing member, respectively.

9. The end-effector assembly according to claim 8, wherein the first constraint portion comprises a first constraint protrusion (36a) disposed on the articulation transmission member, the first constraint protrusion has a concave arc surface matching an outer convex arc surface of the firing member when being most articulated, the second constraint portion comprises a second constraint protrusion (372d) disposed on the articulation joint, and the second constraint protrusion has a convex arc surface matching an inner concave arc surface of the firing member when being most articulated.

10. The end-effector assembly according to claim 8, wherein the first constraint portion comprises a constraint member (68) disposed on the proximal body portion, the constraint member has a concave arc surface (68a) matching an outer convex arc surface of the firing member when being most articulated, the second constraint portion is a convex arc surface (66b) provided at the distal end of the articulation transmission member, and the convex arc surface matching an inner convex arc surface of the firing member when being most articulated.

11. The end-effector assembly according to claim 9 or 10, wherein a receiving slot (351a) is provided in a distal portion of the firing member, and when an articulation angle of the distal execution portion is less than a set value, a partial region of the first constraint portion is received in the receiving slot.

12. The end-effector assembly according to claim 1, wherein the articulation joint comprises a first link (371) and a second link (372), the pivot pin being disposed on the first link and/or the second link, the first link and the second link are connected through two connection pins located on opposite sides of the pivot pin, and one of the connection pins is adapted for the articulation drive pin for cooperating with the articulation transmission member.

13. The end-effector assembly according to claim 1, further comprising an initial position retainer (40) adapted to support the distal execution portion and the proximal body portion so that the distal execution portion is retained in an initial articulated state forming a set angle with respect to the proximal body portion.

14. A surgical instrument (100), comprising a main body portion and an end-effector assembly that is selectively engaged with the main body portion, **characterized in that** the end-effector assembly is the end-effector assembly according to any one of claims 1-13.

## Patentansprüche

1. Eine Endeffektoranordnung (30, 60), umfassend:
einen proximalen Körperabschnitt (30a, 60a), der eine Längsachse (C) definiert; und
einen distalen Ausführungsabschnitt (30b, 60b), der dazu ausgebildet ist, Gewebe zu manipulieren, wobei der distale Ausführungsabschnitt über ein Artikulationsgelenk (30c, 60c), das mit einem Schwenkzapfen (371c) daran versehen ist, schwenkbar mit dem proximalen Körperabschnitt verbunden ist;
der proximale Körperabschnitt ein Artikulationsübertragungselement (36, 66) umfasst, und ein Artikulationsantriebsstift (372a, 672a) an dem Artikulationsgelenk angeordnet ist, wobei das Artikulationsübertragungselement schwenkbar mit dem Artikulationsantriebsstift verbunden ist, und das Artikulationsübertragungselement betreibbar ist, um eine Hin- und Herbewegung entlang der Richtung der Längsachse auszuführen, um den Artikulationsantriebsstift zu betätigen, um um den Schwenkzapfen zu schwenken, und um den distalen Ausführungsabschnitt zu betätigen, um um den Schwenkzapfen zu schwenken; und
wenn sich die Endeffektoranordnung in einer ladebereiten Position befindet, der distale Ausführungsabschnitt relativ zu der Längsachse einen eingeschlossenen Winkel bildet, der nicht gleich 0° ist;
**dadurch gekennzeichnet, dass**
die Endeffektoranordnung ferner einen Begrenzungskanal umfasst, der dazu ausgebildet ist, eine Artikulationsposition eines Auslöseelements (35) während einer Auslösebewegung zu begrenzen, wenn sich der distale Ausführungsabschnitt in einem artikulierten Zustand befindet, wobei der Begrenzungskanal in einem Passbereich zwischen dem proximalen Körperabschnitt und dem Artikulationsgelenk angeordnet ist, und das Auslöseelement in dem Begrenzungskanal angeordnet und in einer Richtung bewegbar ist, in der sich der Begrenzungskanal erstreckt.

2. Die Endeffektoranordnung nach Anspruch 1, wobei, wenn der distale Ausführungsabschnitt betrieben wird, um sich entlang der Richtung der Längsachse zu erstrecken, eine Linie, die einen Achsenmittelpunkt des Artikulationsantriebsstifts und einen Achsenmittelpunkt des Schwenkzapfens verbindet, einen Winkel, der nicht gleich 0° ist, in Bezug auf die Längsachse bildet.

3. Die Endeffektoranordnung nach Anspruch 1, wobei, wenn der distale Ausführungsabschnitt betrieben wird, um sich entlang der Richtung der Längsachse zu erstrecken, der Artikulationsantriebsstift proximal oder distal zu dem Schwenkzapfen angeordnet ist.

4. Die Endeffektoranordnung nach Anspruch 1, wobei, wenn sich die Endeffektoranordnung in der ladebereiten Position befindet, das Artikulationsgelenk geschwenkt ist, so dass eine Linie, die einen Achsenmittelpunkt des Artikulationsantriebsstifts und einen Achsenmittelpunkt des Schwenkzapfens verbindet, senkrecht zu der Längsachse ist.

5. Die Endeffektoranordnung nach Anspruch 1, wobei, wenn das Artikulationsübertragungselement betrieben wird, um sich von einer Startposition zu einer Endposition zu bewegen, der distale Ausführungsabschnitt betätigt wird, um um den Schwenkzapfen zu schwenken und allmählich von der Längsachse weg artikuliert zu werden.

6. Die Endeffektoranordnung nach Anspruch 5, wobei, wenn sich das Artikulationsübertragungselement in der Startposition befindet, der distale Ausführungsabschnitt betrieben wird, um sich entlang der Richtung der Längsachse zu erstrecken.

7. Die Endeffektoranordnung nach Anspruch 5, wobei, wenn sich die Endeffektoranordnung in der ladebereiten Position befindet, das Artikulationsübertragungselement in einer Zwischenposition angeordnet ist, die zwischen der Startposition und der Endposition liegt.

8. Die Endeffektoranordnung nach Anspruch 1, wobei der Begrenzungskanal durch einen ersten Begrenzungsabschnitt und einen zweiten Begrenzungsabschnitt gebildet ist, die jeweils an entgegengesetzten Seiten des Auslöseelements angeordnet sind.

9. Die Endeffektoranordnung nach Anspruch 8, wobei der erste Begrenzungsabschnitt einen ersten Begrenzungsvorsprung (36a) umfasst, der an dem Artikulationsübertragungselement angeordnet ist, der erste Begrenzungsvorsprung eine konkave Bogenfläche aufweist, die bei maximaler Artikulation an eine äußere konvexe Bogenfläche des Auslöseelements angepasst ist, der zweite Begrenzungsabschnitt einen zweiten Begrenzungsvorsprung (372d) umfasst, der an dem Artikulationsgelenk angeordnet ist, und der zweite Begrenzungsvorsprung eine konvexe Bogenfläche aufweist, die bei maximaler Artikulation an eine innere konkave Bogenfläche des Auslöseelements angepasst ist.

10. Die Endeffektoranordnung nach Anspruch 8, wobei der erste Begrenzungsabschnitt ein Begrenzungselement (68) umfasst, das an dem proximalen Körperabschnitt angeordnet ist, das Begrenzungselement eine konkave Bogenfläche (68a) aufweist, die bei maximaler Artikulation an eine äußere konvexe Bogenfläche des Auslöseelements angepasst ist, der zweite Begrenzungsabschnitt eine konvexe Bogenfläche (66b) ist, die an dem distalen Ende des Artikulationsübertragungselements vorgesehen ist, und die konvexe Bogenfläche bei maximaler Artikulation an eine innere konvexe Bogenfläche des Auslöseelements angepasst ist.

11. Die Endeffektoranordnung nach Anspruch 9 oder 10, wobei ein Aufnahmeschlitz (351a) in einem distalen Abschnitt des Auslöseelements vorgesehen ist, und wenn ein Artikulationswinkel des distalen Ausführungsabschnitts kleiner als ein vorgegebener Wert ist, ein Teilbereich des ersten Begrenzungsabschnitts in dem Aufnahmeschlitz aufgenommen ist.

12. Die Endeffektoranordnung nach Anspruch 1, wobei das Artikulationsgelenk ein erstes Verbindungsglied (371) und ein zweites Verbindungsglied (372) umfasst, wobei der Schwenkzapfen an dem ersten Verbindungsglied und/oder dem zweiten Verbindungsglied angeordnet ist, das erste Verbindungsglied und das zweite Verbindungsglied durch zwei Verbindungsstifte verbunden sind, die an entgegengesetzten Seiten des Schwenkzapfens angeordnet sind, und einer der Verbindungsstifte dazu ausgebildet ist, als der Artikulationsantriebsstift mit dem Artikulationsübertragungselement zusammenzuwirken.

13. Die Endeffektoranordnung nach Anspruch 1, ferner umfassend einen Anfangspositionshalter (40), der dazu ausgebildet ist, den distalen Ausführungsabschnitt und den proximalen Körperabschnitt zu stützen, so dass der distale Ausführungsabschnitt in einem anfänglichen artikulierten Zustand gehalten wird, der einen vorgegebenen Winkel in Bezug auf den proximalen Körperabschnitt bildet.

14. Ein chirurgisches Instrument (100), umfassend einen Hauptkörperabschnitt und eine Endeffektoranordnung, die wahlweise mit dem Hauptkörperabschnitt in Eingriff gebracht ist, **dadurch gekennzeichnet, dass** die Endeffektoranordnung die Endeffektoranordnung nach irgendeinem der Ansprüche 1-13 ist.

## Revendications

1. Un ensemble effecteur terminal (30, 60), comprenant :
une portion de corps proximale (30a, 60a) qui définit un axe longitudinal (C) ; et
une portion d'exécution distale (30b, 60b) adaptée pour manipuler un tissu, dans lequel la portion d'exécution distale étant connectée de manière pivotante à la portion de corps proximale par un joint d'articulation (30c, 60c) pourvu d'un axe de pivotement (371c) sur celui-ci ;
la portion de corps proximale comprend un élément de transmission d'articulation (36, 66), et un axe d'entraînement d'articulation (372a, 672a) est agencé sur le joint d'articulation, dans lequel l'élément de transmission d'articulation est connecté de manière pivotante à l'axe d'entraînement d'articulation, et l'élément de transmission d'articulation est apte à effectuer un mouvement de va-et-vient le long de la direction de l'axe longitudinal pour actionner l'axe d'entraînement d'articulation afin qu'il pivote autour de l'axe de pivotement et pour actionner la portion d'exécution distale afin qu'elle pivote autour de l'axe de pivotement ; et
lorsque l'ensemble effecteur terminal est dans une position prête à être chargée, la portion d'exécution distale forme un angle inclus non égal à 0° par rapport à l'axe longitudinal ;
**caractérisé en ce que**
l'ensemble effecteur terminal comprend en outre un canal de contrainte adapté pour limiter une position d'articulation d'un élément de déclenchement (35) pendant un mouvement de déclenchement lorsque la portion d'exécution distale est dans un état articulé, le canal de contrainte étant agencé dans une région d'ajustement entre la portion de corps proximale et le joint d'articulation, et l'élément de déclenchement étant agencé dans le canal de contrainte et mobile dans une direction dans laquelle le canal de contrainte s'étend.

2. L'ensemble effecteur terminal selon la revendication 1, dans lequel lorsque la portion d'exécution distale est actionnée pour s'étendre le long de la direction de l'axe longitudinal, une ligne reliant un centre d'axe de l'axe d'entraînement d'articulation et un centre d'axe de l'axe de pivotement forme un angle non égal à 0° par rapport à l'axe longitudinal.

3. L'ensemble effecteur terminal selon la revendication 1, dans lequel lorsque la portion d'exécution distale est actionnée pour s'étendre le long de la direction de l'axe longitudinal, l'axe d'entraînement d'articulation est situé proximalement ou distalement par rapport à l'axe de pivotement.

4. L'ensemble effecteur terminal selon la revendication 1, dans lequel lorsque l'ensemble effecteur terminal est dans la position prête à être chargée, le joint d'articulation est pivoté de sorte qu'une ligne reliant un centre d'axe de l'axe d'entraînement d'articulation et un centre d'axe de l'axe de pivotement est perpendiculaire à l'axe longitudinal.

5. L'ensemble effecteur terminal selon la revendication 1, dans lequel lorsque l'élément de transmission d'articulation est actionné pour se déplacer d'une position de départ à une position d'extrémité, la portion d'exécution distale est actionnée pour pivoter autour de l'axe de pivotement et pour être progressivement articulée à l'écart de l'axe longitudinal.

6. L'ensemble effecteur terminal selon la revendication 5, dans lequel lorsque l'élément de transmission d'articulation est dans la position de départ, la portion d'exécution distale est actionnée pour s'étendre le long de la direction de l'axe longitudinal.

7. L'ensemble effecteur terminal selon la revendication 5, dans lequel lorsque l'ensemble effecteur terminal est dans la position prête à être chargée, l'élément de transmission d'articulation est situé dans une position intermédiaire qui est entre la position de départ et la position d'extrémité.

8. L'ensemble effecteur terminal selon la revendication 1, dans lequel le canal de contrainte est formé par une première portion de contrainte et une deuxième portion de contrainte agencées sur des côtés opposés de l'élément de déclenchement, respectivement.

9. L'ensemble effecteur terminal selon la revendication 8, dans lequel la première portion de contrainte comprend une première saillie de contrainte (36a) disposée sur l'élément de transmission d'articulation, la première saillie de contrainte présente une surface en arc concave correspondant à une surface en arc convexe extérieure de l'élément de déclenchement dans l'état le plus articulé, la deuxième portion de contrainte comprend une deuxième saillie de contrainte (372d) disposée sur le joint d'articulation, et la deuxième saillie de contrainte présente une surface en arc convexe correspondant à une surface en arc concave intérieure de l'élément de déclenchement dans l'état le plus articulé.

10. L'ensemble effecteur terminal selon la revendication 8, dans lequel la première portion de contrainte comprend un élément de contrainte (68) disposé sur la portion de corps proximale, l'élément de contrainte présente une surface en arc concave (68a) correspondant à une surface en arc convexe extérieure de l'élément de déclenchement dans l'état le plus articulé, la deuxième portion de contrainte est une surface en arc convexe (66b) prévue à l'extrémité distale de l'élément de transmission d'articulation, et la surface en arc convexe correspond à une surface en arc convexe intérieure de l'élément de déclenchement dans l'état le plus articulé.

11. L'ensemble effecteur terminal selon la revendication 9 ou 10, dans lequel une fente de réception (351a) est prévue dans une portion distale de l'élément de déclenchement, et lorsque l'angle d'articulation de la portion d'exécution distale est inférieur à une valeur définie, une région partielle de la première portion de contrainte est reçue dans la fente de réception.

12. L'ensemble effecteur terminal selon la revendication 1, dans lequel le joint d'articulation comprend un premier élément de liaison (371) et un deuxième élément de liaison (372), l'axe de pivotement étant disposé sur le premier élément de liaison et/ou le deuxième élément de liaison, le premier élément de liaison et le deuxième élément de liaison sont connectés par deux axes de connexion situés sur des côtés opposés de l'axe de pivotement, et l'un des axes de connexion est adapté à l'axe d'entraînement d'articulation pour coopérer avec l'élément de transmission d'articulation.

13. L'ensemble effecteur terminal selon la revendication 1, comprenant en outre un dispositif de retenue de position initiale (40) adapté pour supporter la portion d'exécution distale et la portion de corps proximale de sorte que la portion d'exécution distale soit retenue dans un état articulé initial formant un angle défini par rapport à la portion de corps proximale.

14. Un instrument chirurgical (100), comprenant une portion de corps principal et un ensemble effecteur terminal qui est sélectivement engagé avec la portion de corps principal, **caractérisé en ce que** l'ensemble effecteur terminal est l'ensemble effecteur terminal selon l'une quelconque des revendications 1-13.
